# FASCICULE DE BREVET EUROPEEN

(11) **EP 4 062 823 B1**
(45) Date de publication et mention de la délivrance du brevet: **18.10.2023**
(21) Numéro de dépôt: 22163537.8
(22) Date de dépôt: 22.03.2022
(51) Int. Cl.: A61B 5/00, A45D 44/00

(54) **DISPOSITIF DE DÉTERMINATION DE BRILLANCE**
VORRICHTUNG ZUR BESTIMMUNG DER HELLIGKEIT
DEVICE FOR DETERMINING BRIGHTNESS

(30) Priorité: 23.03.2021 FR 2102924
(43) Date de publication de la demande: 28.09.2022
(73) Titulaire: SEB S.A., 69130 Ecully (FR)
(72) Inventeur: PLANTEROSE, Thierry, 69230 Saint-Genis-Laval (FR); SABATTIER, Johan, 69370 Saint-Didier-au-Mont-d'Or (FR); COMPARD, Stéphanie, 69330 MEYZIEU (FR); RENAULT, Fabrice, 69680 Chassieu (FR); YOUSIF, Riva, 69120 VAULX-EN-VELIN (FR)
(74) Mandataire: Germain Maureau

(56) Documents cités:
- WO-A1-2017/032636
- WO-A1-2020/125923
- JP-A- 2004 198 398
- US-A1- 2005 211 599
- US-A1- 2012 320 191

## Description

### Domaine technique

La présente invention se rapporte à un dispositif de détermination de brillance configuré pour déterminer une brillance relative d'une pluralité de fibres capillaires.

### Etat de la technique

De manière connue, le document WO2017001229A1 concerne un dispositif de mesure de rugosité et de brillance de cheveux, comprenant :
a) une première zone comprenant un gradient ascendant de brillance, et
b) une seconde zone comprenant un gradient ascendant de rugosité, la
première zone étant alignée avec la seconde zone, et le gradient ascendant de rugosité étant fournie par un motif texturé qui est texturé pour imiter des cheveux.

Toutefois, ces solutions ne donnent pas une entière satisfaction.

En effet, le dispositif du document WO2017001229A1 permet de comparer des cheveux avec un gradient mais ne permet pas d'estimer automatiquement la brillance des cheveux. D'autres dispositifs permettant d'estimer la brillance des cheveux sont connus des documents US2012320191 A1 et WO2017032636 A1.

La présente invention a pour but de résoudre tout ou partie des inconvénients mentionnés ci-dessus.

### Résumé de l'invention

A cet effet, la présente invention concerne un dispositif de détermination de brillance configuré pour déterminer une brillance relative d'une pluralité de fibres capillaires, ou plus particulièrement d'une mèche de cheveux, dans lequel le dispositif de détermination de brillance comprend :
- une surface de réception configurée pour recevoir la pluralité de fibres capillaires, ou plus particulièrement la mèche de cheveux ;
- un élément optique de mesure configuré pour déterminer la brillance relative des fibres capillaires reçues sur la surface de réception;
- un élément d'alignement configuré pour aligner la pluralité de fibres capillaires, ou plus particulièrement la mèche de cheveux, lorsque la pluralité de fibres capillaires, ou plus particulièrement la mèche de cheveux, est positionnée sur la surface de réception ;

- un élément de mise sous tension configuré pour mettre sous tension la pluralité de fibres capillaires, ou plus particulièrement la mèche de cheveux, sur ladite surface de réception;
- un élément d'occultation configuré pour occulter au moins partiellement l'élément optique de mesure de la lumière ambiante lorsque la pluralité de fibres capillaires, ou plus particulièrement la mèche de cheveux, est positionnée sur la surface de réception.

Selon un mode de réalisation, l'élément optique de mesure est configuré pour déterminer la brillance relative des fibres capillaires par émission de lumière sur les fibres capillaires et mesure de faisceaux lumineux réfléchis et diffus.

Un tel dispositif permet d'obtenir une détermination de la brillance relative des fibres capillaires plus précise. En effet, l'élément optique de mesure étant protégé de la lumière ambiante par l'élément d'occultation, la mesure de la brillance obtenue est d'autant plus précise.

Un dispositif de détermination de brillance comprenant un tel élément de mise sous tension permet un bon maintien des fibres capillaires et donc une meilleure détermination de la brillance. En effet, la détermination de la brillance étant effectuée par émission de lumière, il est important que les fibres capillaires soient maintenues sous tension, de manière à ce que la normale de la surface de réception soit sensiblement la même pour toutes les positions des rayons de lumière émis par l'élément optique de mesure, et éviter ainsi une différence angulaire après réflexion.

Un dispositif comprenant un élément d'alignement permet d'aligner les fibres capillaires de la pluralité de fibres capillaires et ainsi obtenir une détermination de la brillance relative plus précise.

Le dispositif de détermination de brillance peut en outre présenter une ou plusieurs des caractéristiques suivantes, prises seules ou en combinaison.

Selon un mode de réalisation, l'élément de mise sous tension est disposé sur l'élément d'occultation. Une telle disposition permet d'obtenir un dispositif de détermination de brillance plus compact.

Dans le dispositif de la présente invention, l'élément d'occultation est mobile entre une position ouverte, dans laquelle l'élément optique de mesure n'est pas protégé de la lumière ambiante par l'élément d'occultation, et une position fermée, dans laquelle l'élément optique de mesure est protégé de la lumière ambiante par l'élément d'occultation.

Une telle disposition permet de disposer aisément les fibres capillaires sur la surface de réception lorsque l'élément d'occultation est dans la position ouverte tout en permettant une détermination précise de la brillance des fibres capillaires lorsque l'élément d'occultation est dans la position fermée.

Selon un mode de réalisation l'élément d'occultation libère l'accès à l'élément optique de mesure lors du passage de la position fermée à la position ouverte et couvre au moins partiellement l'élément optique de mesure en position fermée, protégeant ainsi ledit élément optique de mesure de la lumière ambiante.

Selon un mode de réalisation, l'élément d'occultation est mobile en rotation autour d'un axe de rotation.

Selon un mode de réalisation, l'élément d'occultation est mobile selon l'axe de rotation entre la position ouverte et la position fermée.

Selon un mode de réalisation, le dispositif de détermination de brillance comprend un élément d'actionnement mobile entre une première position et une deuxième position, l'élément d'actionnement étant configuré pour entrainer un déplacement de l'élément d'occultation entre la position fermée et la position ouverte lorsque l'élément d'actionnement est déplacé entre la première position et la deuxième position.

Selon un mode de réalisation, l'élément d'actionnement est mobile en translation selon un axe de translation. Une telle disposition permet d'obtenir un dispositif de détermination de brillance plus compact.

Selon un mode de réalisation, l'axe de translation de l'élément d'actionnement est perpendiculaire à l'axe de rotation.

Selon un mode de réalisation, l'élément d'actionnement comprend au moins une crémaillère configurée pour coopérer avec au moins un secteur de roue dentée prévue sur l'élément d'occultation.

Selon un mode de réalisation, le dispositif de détermination de brillance comprend un élément de préhension fixé à la crémaillère de l'élément d'actionnement. Une telle disposition permet à un utilisateur d'actionner l'élément d'actionnement facilement.

Selon un mode de réalisation, l'élément de préhension comprend une pluralité de rainures perpendiculaires à l'axe de translation. Une telle disposition permet une bonne préhension.

Selon un mode de réalisation, le dispositif de détermination de brillance comprend deux éléments d'actionnement comprenant chacun une crémaillère configurée pour coopérer avec au moins un secteur de roue dentée respectif prévu sur l'élément d'occultation. Le dispositif de détermination de brillance comprend également deux éléments de préhension fixés chacun à une crémaillère d'un élément d'actionnement respectif. De façon avantageuse, les éléments de préhension et les éléments d'actionnement sont situés sur des bords latéraux opposés du dispositif de détermination de brillance. Une telle disposition permet à un utilisateur d'utiliser le dispositif de détermination de brillance de la main droite ou de la main gauche avec la même aisance.

Selon un mode de réalisation, le dispositif de détermination de brillance comprend un élément de rappel configuré pour rappeler l'élément d'occultation vers la position fermée. Une telle disposition permet de maintenir l'élément d'occultation en position fermée et ainsi assurer une détermination précise de la brillance relative d'une pluralité de fibres capillaires.

Selon un mode de réalisation, l'élément de rappel peut être de toute sorte telle qu'un ressort ou une lame par exemple.

Selon un mode de réalisation, le dispositif de détermination de brillance comprend un élément de blocage configuré pour bloquer l'élément d'occultation en position ouverte.

Selon un mode de réalisation, l'élément de blocage comprend une unité de blocage configurée pour pénétrer dans un orifice de blocage prévu sur l'élément de préhension lorsque l'élément d'occultation est en position ouverte. Une telle disposition permet d'empêcher la translation de l'élément de préhension, et par la même de la crémaillère, ce qui a pour effet de bloquer l'élément d'occultation en position ouverte. Il est cependant possible de libérer l'élément d'occultation de sa position ouverte en faisant sortir l'unité de blocage de l'orifice de blocage.

Selon un mode de réalisation, l'élément de blocage comprend une unité de pression configurée pour appliquer une force sur l'unité de blocage de sorte que l'unité de blocage pénètre dans l'orifice de blocage. Une telle disposition permet d'obtenir un élément d'occultation automatiquement bloqué en position ouverte. Selon un mode de réalisation, l'unité de pression peut-être de toute sorte telle qu'un ressort ou une lame par exemple.

Selon un mode de réalisation, l'élément d'occultation est amovible. Une telle disposition permet de faciliter le nettoyage du dispositif de détermination de brillance.

Selon un mode de réalisation, le dispositif de détermination de brillance comprend un corps principal sur lequel est monté l'élément optique de mesure, l'élément d'occultation comprenant un premier élément de fixation configuré pour coopérer avec un deuxième élément de fixation prévu sur le corps principal de sorte que ledit élément d'occultation soit fixé de manière amovible au corps principal.

Selon un mode de réalisation, le premier élément de fixation est un élément de fixation femelle et le deuxième élément de fixation est un élément de fixation mâle.

Selon un mode de réalisation, le premier élément de fixation est un orifice de fixation et le deuxième élément de fixation est un doigt de fixation. Une telle disposition permet d'obtenir un élément d'occultation amovible facilement détachable et rattachable.

Selon un mode de réalisation, l'élément d'occultation est fixé de manière amovible à l'aide d'une paire de premiers éléments de fixation et d'une paire de deuxièmes éléments de fixation. Une telle disposition permet d'obtenir une fixation de l'élément d'occultation avec un seul degré de liberté.

Selon un mode de réalisation, l'élément d'occultation comprend un premier aimant de fixation configuré pour coopérer avec un deuxième aimant de fixation de sorte que ledit élément d'occultation soit fixé au reste du dispositif de détermination de brillance magnétiquement. Une telle disposition permet également d'obtenir un élément d'occultation amovible facilement détachable et rattachable.

Selon un mode de réalisation, le premier aimant de fixation et le deuxième aimant de fixation sont des aimants permanents.

Selon un mode de réalisation, l'élément d'alignement est un peigne configuré pour recevoir et aligner la pluralité de fibres capillaires, ou plus particulièrement la mèche de cheveux.

Selon un mode de réalisation, l'élément d'alignement comprend au moins une fente d'alignement configurée pour recevoir et aligner la pluralité de fibres capillaires, ou plus particulièrement la mèche de cheveux.

Selon un mode de réalisation, le dispositif de détermination de brillance comprend un canal d'insertion configuré pour faciliter l'insertion de la pluralité de fibres capillaires dans l'au moins une fente d'alignement. Un tel canal d'insertion permet de créer un effet détrompeur pour l'utilisateur, ce qui permet de s'assurer que la pluralité de fibres capillaires est reçue selon une orientation prédéterminée.

Selon un mode de réalisation, le corps principal du dispositif comprend également au moins un élément détrompeur, tel qu'au moins un bouton par exemple. Une telle disposition permet de créer un effet détrompeur pour l'utilisateur, ce qui permet de s'assurer que la pluralité de fibres capillaires est reçue selon l'orientation prédéterminée.

Selon un mode de réalisation, l'élément de mise sous tension comprend une membrane incurvée.

La membrane incurvée permet de conférer deux points de contacts avec la pluralité de fibres capillaires pour les tendre sur la surface de réception lorsque l'utilisateur va déplacer le dispositif de détermination de brillance.

Selon un mode de réalisation, l'élément de mise sous tension comprend au moins un patin de maintien.

Une telle disposition permet de faire en sorte que la pluralité de fibres capillaires soit tendue sur la surface de réception lorsque l'utilisateur va déplacer le dispositif de détermination de brillance.

Selon un mode de réalisation, l'élément de mise sous tension est au moins partiellement souple. Grâce à l'utilisation d'au moins un élément de tension au moins partiellement souple, il est également possible à l'utilisateur d'assurer que les fibres capillaires, ou la mèche de cheveux, soient tendues et davantage alignées sur la surface de réception en appliquant une force de traction sur les fibres capillaires de sorte à créer une tension des fibres capillaires, ou de la mèche de cheveux, sur l'au moins un patin de maintien souple. En effet, l'au moins un patin de maintien souple va limiter les déplacements des fibres capillaires, ou de la mèche de cheveux, et permettre ainsi d'obtenir des fibres capillaires, ou une mèche de cheveux, tendues et davantage alignées sur la surface de réception. Des fibres capillaires, ou une mèche de cheveux, tendues et davantage alignées sur la surface de réception permet une mesure plus précise de la brillance.

Selon un mode de réalisation, l'au moins un patin de maintien ou la membrane incurvée comprend une surface de contact ayant un coefficient de frottement élevé compris entre 30 et 70 ShA et préférablement 60 ShA, de sorte à maintenir les fibres capillaires, ou la mèche de cheveux, en position sans les casser.

Selon un mode de réalisation, l'élément de mise sous tension comprend deux patins de maintien disposés de part et d'autre de l'élément optique de mesure lorsque l'élément d'occultation est dans une position fermée.

Selon un mode de réalisation, la surface de réception comprend des logements, tels que des renfoncements, configurés pour recevoir les patins de maintien lorsque l'élément d'occultation est dans la position fermée.

L'invention concerne également un appareil de traitement de fibres capillaires, ou plus particulièrement d'une mèche de cheveux, comprenant un dispositif de détermination de brillance selon l'invention.

Au sens de la présente invention, un appareil de traitement de fibres capillaires peut être de toute sorte tel qu'une brosse à cheveux, un fer à lisser ou encore un fer à friser par exemple.

Selon un mode de réalisation, l'appareil de traitement de fibres capillaires est un appareil de traitement cosmétique de fibres capillaires.

Les différents aspects définis ci-dessus non incompatibles peuvent être combinés.

### Brève description des figures

On comprendra mieux les buts, aspects et avantages de la présente invention, d'après la description donnée ci-après d'un mode particulier de réalisation de l'invention présenté à titre d'exemple non limitatif, en se référant aux dessins annexés dans lesquels :
[Fig 1] représente une vue en perspective d'un dispositif de détermination de brillance, selon un premier mode de réalisation de l'invention ;
[Fig 2] représente une vue partielle en perspective du dispositif de détermination de brillance de la figure 1 dans laquelle un élément d'occultation amovible est non représenté ;
[Fig 3] représente une vue éclatée en perspective du dispositif de détermination de brillance de la figure 1 ;
[Fig 4] représente une vue éclatée en perspective du dispositif de détermination de brillance de la figure 1 ;
[Fig 5] représente une vue en perspective d'un dispositif de détermination de brillance selon un deuxième mode de réalisation de l'invention et dans une première configuration ;
[Fig 6] représente une vue partielle en perspective du dispositif de détermination de brillance de la figure 5 dans une deuxième configuration ; et
[Fig 7] représente une vue en perspective d'un dispositif de traitement de fibres capillaires comprenant le dispositif de détermination de brillance de la figure 5.

La présente invention concerne un dispositif de détermination de brillance configuré pour déterminer une brillance relative d'une pluralité de fibres capillaires, ou plus particulièrement d'une mèche de cheveux. Le dispositif de détermination de brillance comprend une surface de réception 12 configurée pour recevoir la pluralité de fibres capillaires, ou plus particulièrement la mèche de cheveux, ainsi qu'un élément optique de mesure 14 configuré pour déterminer la brillance relative des fibres capillaires reçues sur la surface de réception 12. L'élément optique de mesure 14 est configuré pour déterminer la brillance relative des fibres capillaires par émission de lumière sur les fibres capillaires et mesure de faisceaux lumineux réfléchis et diffus. L'élément optique de mesure 14 peut être de tout type, tel que celui divulgué dans la demande de brevet FR2012781 au nom de la demanderesse.

Le dispositif de détermination de brillance comprend également un élément d'alignement 16 configuré pour aligner la pluralité de fibres capillaires, ou plus particulièrement la mèche de cheveux, lorsque la pluralité de fibres capillaires, ou plus particulièrement la mèche de cheveux, est positionnée sur la surface de réception 12. Un dispositif de détermination de brillance comprenant un élément d'alignement 16 permet d'aligner les fibres capillaires de la pluralité de fibres capillaires et ainsi d'obtenir une détermination de la brillance relative plus précise.

Le dispositif de détermination de brillance comprend en outre un élément de mise sous tension 18 qui est configuré pour mettre sous tension la pluralité de fibres capillaires, ou plus particulièrement la mèche de cheveux, sur ladite surface de réception 12. Un dispositif de détermination de brillance comprenant un tel élément de mise sous tension 18 permet un bon maintien des fibres capillaires et donc une meilleure détermination de la brillance. En effet, la détermination de la brillance étant effectuée par émission de lumière, il est important que les fibres capillaires soient maintenues sous tension, de manière à ce que la normale de la surface de réception 12 soit la même pour toutes les positions des rayons de lumière émis par l'élément optique de mesure 14, et éviter ainsi une différence angulaire après réflexion.

Le dispositif de détermination de brillance comprend également un élément d'occultation 20 configuré pour occulter au moins partiellement l'élément optique de mesure 14 de la lumière ambiante lorsque la pluralité de fibres capillaires, ou plus particulièrement la mèche de cheveux, est positionnée sur la surface de réception 12. Un tel dispositif de détermination de brillance permet d'obtenir une détermination de la brillance relative des fibres capillaires plus précise. En effet, l'élément optique de mesure 14 étant protégé de la lumière ambiante par l'élément d'occultation 20, la mesure de la brillance obtenue est d'autant plus précise.

De façon avantageuse, l'élément de mise sous tension 18 est disposé sur l'élément d'occultation 20. Une telle disposition permet d'obtenir un dispositif de détermination de brillance plus compact.

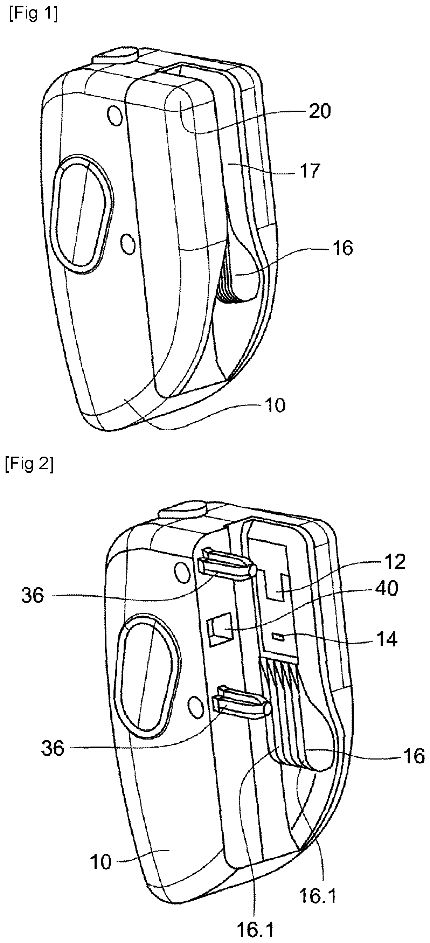

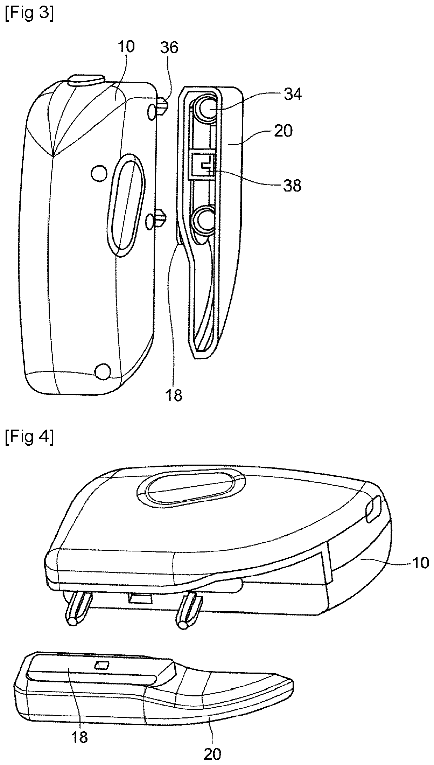

Selon un premier mode de réalisation illustré aux figures 1 à 4, l'élément d'occultation 20 est amovible, ce qui permet de faciliter le nettoyage du dispositif de détermination de brillance. Néanmoins, selon une variante de réalisation ne faisant pas partie de l'invention, l'élément d'occultation 20 pourrait être fixe

Dans ce premier mode de réalisation, le dispositif de détermination de brillance comprend un corps principal 10 sur lequel est monté l'élément optique de mesure 14, et l'élément d'occultation 20 comprend deux premiers éléments de fixation 34, par exemple femelle, chacun d'eux étant configuré pour coopérer respectivement avec un deuxième élément de fixation 36, par exemple mâle, prévu sur le corps principal 10 de sorte que ledit élément d'occultation 20 soit fixé de manière amovible au corps principal 10. Une telle disposition permet d'obtenir un élément d'occultation 20 qui est amovible, facilement détachable et rattachable et ayant un seul degré de liberté.

Dans ce premier mode de réalisation, l'élément d'occultation 20 comprend un premier aimant de fixation 38, qui est avantageusement un aimant permanent, configuré pour coopérer avec un deuxième aimant de fixation 40, qui est avantageusement un aimant permanent, de sorte que ledit élément d'occultation 20 soit fixé au corps principal 10 magnétiquement. Une telle disposition permet d'obtenir un élément d'occultation 20 amovible facilement détachable et rattachable.

Selon le premier mode de réalisation de l'invention, l'élément d'alignement 16 comprend au moins une fente d'alignement 16.1 ou une pluralité de fentes d'alignement 16.1 configurée pour recevoir et aligner la pluralité de fibres capillaires, ou plus particulièrement la mèche de cheveux. Les fentes d'alignement 16.1 forment avantageusement un peigne. De façon avantageuse, le dispositif de détermination de brillance comprend un canal d'insertion 17 configuré pour faciliter l'insertion de la pluralité de fibres capillaires dans la fente d'alignement. Le canal d'insertion 17 peut par exemple s'étendre sensiblement parallèlement à la fente d'alignement 16.1 ou aux fentes d'alignement 16.1, et peut par exemple déboucher dans une paroi latérale du dispositif de détermination de brillance. Un tel canal d'insertion 17 permet de créer un effet détrompeur pour l'utilisateur, ce qui permet de s'assurer que la pluralité de fibres capillaires est reçue selon une orientation prédéterminée.

Selon le premier mode de réalisation de l'invention, l'élément de mise sous tension 18 est situé en regard de la surface de réception 12 et de la ou des fente(s) d'alignement 16.1, et comprend une unique membrane incurvée qui est avantageusement partiellement souple. Grâce à l'utilisation d'une membrane incurvée, il est également possible à l'utilisateur d'assurer que les fibres capillaires, ou la mèche de cheveux, soient tendues et davantage alignées sur la surface de réception 12 en appliquant une force de traction sur les fibres capillaires de sorte à créer une tension des fibres, ou de la mèche de cheveux, capillaires sur la membrane incurvée. En effet, la membrane incurvée va limiter les déplacements des fibres capillaires, ou de la mèche de cheveux, et permettre ainsi d'obtenir des fibres capillaires, ou une mèche de cheveux, tendues et davantage alignées sur la surface de réception 12. Des fibres capillaires, ou une mèche de cheveux, tendues et davantage alignées sur la surface de réception 12 permet une mesure plus précise de la brillance.

La membrane incurvée comprend avantageusement une surface de contact ayant un coefficient de frottement élevé compris entre 30 et 70 ShA et préférablement 60 ShA, de sorte à maintenir les fibres capillaires, ou la mèche de cheveux, en position sans les casser.

Un procédé pour déterminer la brillance relative d'une pluralité de fibres capillaires à l'aide d'un dispositif de détermination de brillance selon le premier mode de réalisation comprend les étapes suivantes :
- introduire des fibres capillaires dans le canal d'insertion 17,
- positionner la pluralité de fibres capillaires sur la surface de réception 12, et occulter au moins partiellement l'élément optique de mesure 14 de la lumière ambiante à l'aide de l'élément d'occultation 20,

- déplacer le dispositif de détermination de brillance en direction des pointes de la pluralité de fibres capillaires de manière à aligner la pluralité de fibres capillaires à l'aide de l'élément d'alignement 16, et à mettre sous tension la pluralité de fibres capillaires à l'aide de l'élément de mise sous tension 18, et
- déterminer la brillance relative des fibres capillaires à l'aide l'élément optique de mesure 14,
- transmettre sur une interface utilisateur une information sur la brillance relative ainsi déterminée.

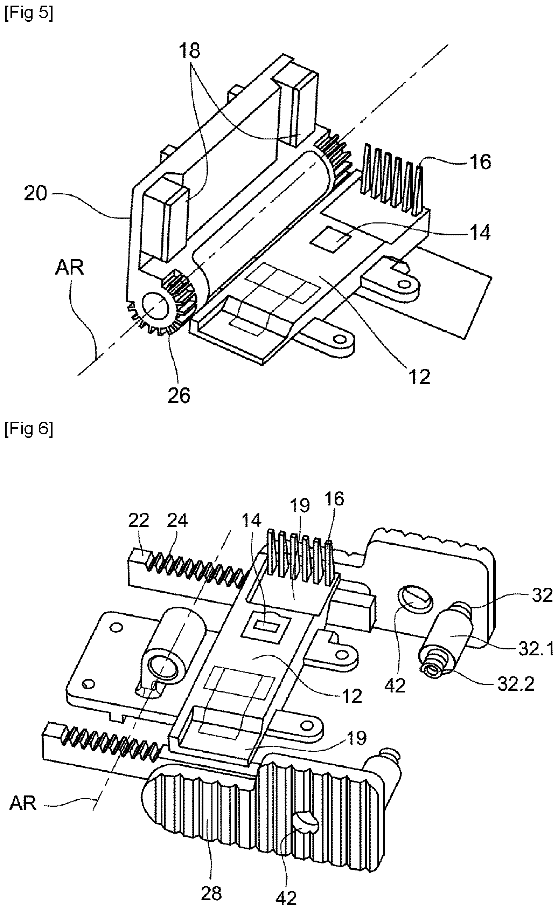

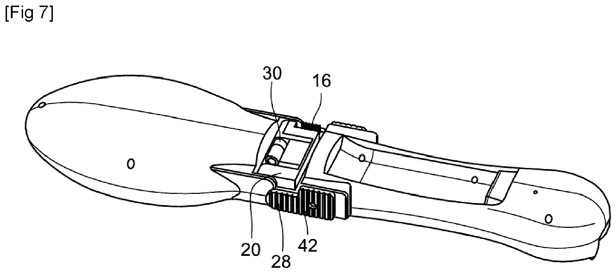

Selon un second mode de réalisation illustré aux figures 5 à 6, l'élément d'occultation 20 est mobile, et par exemple mobile en rotation autour d'un axe de rotation AR, entre une position ouverte (voir la figure 5), dans laquelle l'élément optique de mesure 14 n'est pas protégé de la lumière ambiante par l'élément d'occultation 20, et une position fermée (voir la figure 7), dans laquelle l'élément optique de mesure 14 est protégé de la lumière ambiante par l'élément d'occultation 20. Une telle disposition permet à un utilisateur de disposer ses fibres capillaires sur la surface de réception 12 lorsque l'élément d'occultation 20 est dans la position ouverte, de passer l'élément d'occultation 20 dans la position fermée pour limiter les mouvements des fibres capillaires tout en protégeant l'élément optique de mesure 14 de la lumière ambiante. Plus précisément, l'élément d'occultation 20 libère l'accès à l'élément optique de mesure 14 lors du passage de la position fermée à la position ouverte et couvre au moins partiellement l'élément optique de mesure 14 en position fermée, protégeant ainsi ledit élément optique de mesure 14 de la lumière ambiante.

Selon le second mode de réalisation illustré aux figures 5 à 6, le dispositif de détermination de brillance comprend de plus un élément d'actionnement 22 mobile en translation selon un axe de translation perpendiculaire à l'axe de rotation AR entre une première position et une deuxième position, l'élément d'actionnement 22 étant configuré pour entrainer un déplacement de l'élément d'occultation 20 entre la position fermée et la position ouverte lorsque l'élément d'actionnement 22 est déplacé entre la première position et la deuxième position. Une telle disposition permet d'obtenir un dispositif de détermination de brillance plus compact. L'élément d'actionnement 22 comprend avantageusement au moins une crémaillère 24 configurée pour coopérer avec au moins un secteur de roue dentée 26 prévue sur l'élément d'occultation 20.

Le dispositif de détermination de brillance comprend de plus un élément de préhension 28 fixé à la crémaillère 24 de l'élément d'actionnement 22. Une telle disposition permet à un utilisateur d'actionner l'élément d'actionnement 22 facilement. L'élément de préhension 28 comprend par exemple une pluralité de rainures perpendiculaires à l'axe de translation. Une telle disposition permet d'obtenir un élément de préhension 28 ayant une bonne préhension.

Selon le second mode de réalisation de l'invention, l'élément d'alignement 16 est un peigne configuré pour recevoir et aligner la pluralité de fibres capillaires, ou plus particulièrement la mèche de cheveux.

Dans ce second mode de réalisation, l'élément de mise sous tension 18 comprend deux patins de maintien qui sont au moins partiellement souples et qui sont disposés de part et d'autre de l'élément optique de mesure 14 lorsque l'élément d'occultation 20 est dans une position fermée. Les deux patins de maintien peuvent par exemple être solidaires de l'élément d'occultation 20. De façon avantageuse, la surface de réception 12 comprend deux logements 19 configurés pour recevoir les patins de maintien lorsque l'élément d'occultation 20 est dans une position fermée.

Selon un mode de réalisation préféré du second mode de réalisation, le dispositif de détermination de brillance comprend deux éléments d'actionnement 22 comprenant chacun une crémaillère 24 configurée pour coopérer avec au moins un secteur de roue dentée 26 respectif prévu sur l'élément d'occultation 20. Le dispositif de détermination de brillance comprend également deux éléments de préhension 28 fixés chacun à une crémaillère 24 d'un élément d'actionnement 22 respectif. De façon avantageuse, les éléments de préhension 28 et les éléments d'actionnement 22 sont situés sur des bords latéraux opposés du dispositif de détermination de brillance. Une telle disposition permet à un utilisateur d'utiliser le dispositif de détermination de brillance de la main droite ou de la main gauche avec la même aisance.

Dans ce second mode de réalisation, le dispositif de détermination de brillance comprend un élément de rappel 30, en l'espèce un ressort, configuré pour rappeler l'élément d'occultation 20 vers la position fermée. Une telle disposition permet de maintenir l'élément d'occultation 20 en position fermée et ainsi d'assurer une détermination précise de la brillance relative d'une pluralité de fibres capillaires.

Le dispositif de détermination de brillance comprend avantageusement un élément de blocage 32 configuré pour bloquer l'élément d'occultation 20 en position ouverte. L'élément de blocage 32 comprend par exemple une unité de blocage 32.1 configurée pour pénétrer dans un orifice de blocage 42 prévu sur l'élément de préhension 28 lorsque l'élément d'occultation 20 est en position ouverte. Une telle disposition permet d'empêcher la translation de l'élément de préhension 28, et par la même de la crémaillère 24, ce qui a pour effet de bloquer l'élément d'occultation 20 en position ouverte. Il est cependant possible de libérer l'élément d'occultation 20 de sa position ouverte en faisant sortir l'unité de blocage 32.1 hors de l'orifice de blocage 42. L'élément de blocage 32 comprend également une unité de pression 32.2, en l'espèce un ressort, configurée pour appliquer une force de poussée sur l'unité de blocage 32.1 de sorte que l'unité de blocage 32.1 pénètre dans l'orifice de blocage 42 lorsque l'unité de blocage 32.1 est située en regard de l'orifice de blocage 42. Une telle disposition permet d'obtenir un élément d'occultation 20 automatiquement bloqué en position ouverte.

Un procédé pour déterminer la brillance relative d'une pluralité de fibres capillaires à l'aide d'un dispositif de détermination de brillance selon le second mode de réalisation comprend les étapes suivantes :
- positionner l'élément d'occultation 20 dans la position ouverte,
- positionner la pluralité de fibres capillaires sur la surface de réception 12 et aligner la pluralité de fibres capillaires à l'aide de l'élément d'alignement 16,
- déplacer l'élément d'occultation 20 dans la position fermée de manière à occulter au moins partiellement l'élément optique de mesure 14 de la lumière ambiante à l'aide de l'élément d'occultation 20,

- déplacer le dispositif de détermination de brillance en direction des pointes de la pluralité de fibres capillaires de manière à aligner la pluralité de fibres capillaires à l'aide de l'élément d'alignement 16, et à mettre sous tension la pluralité de fibres capillaires à l'aide de l'élément de mise sous tension 18,
- déplacer le dispositif de détermination de brillance en direction des pointes de la pluralité de fibres capillaires,
- déterminer la brillance relative des fibres capillaires à l'aide l'élément optique de mesure 14, et
- transmettre sur une interface utilisateur une information sur la brillance relative ainsi déterminée.

Que ce soit dans le premier ou le deuxième mode de réalisation, à partir de l'information transmise, l'utilisateur pourra ajuster la brillance relative des fibres capillaires par l'application d'un traitement capillaire adapté.

En outre, ce traitement pourra lui être recommandé via une interface utilisateur sur la base des mesures de brillance relative réalisées au cours du temps et d'un profil utilisateur.

Ce profil utilisateur pourra comprendre des données déclarées par l'utilisateur et/ou des données déterminées par d'autres dispositifs de mesure.

Ces données peuvent par exemple concerner l'âge, la couleur de peau, la couleur de cheveux, la longueur de cheveux, l'épaisseur des cheveux, la date du dernier lavage, une information sur la production de sébum, ...

L'invention concerne également un appareil de traitement de fibres capillaires, ou plus particulièrement d'une mèche de cheveux, comprenant un dispositif de détermination de brillance selon le second mode de réalisation. Un tel appareil de traitement de fibres capillaires est représenté à la figure 7. Au sens de la présente invention, un appareil de traitement de fibres capillaires peut être de toute sorte tel qu'une brosse à cheveux, un fer à lisser ou encore un fer à friser par exemple. Selon un mode de réalisation, le dispositif de traitement de fibres capillaires est un dispositif de traitement cosmétique de fibres capillaires.

Bien entendu, l'invention n'est pas limitée aux modes de réalisation représentés et décrits ci-avant, mais en couvre au contraire toutes les variantes.

En particulier, dans le mode d réalisation illustré aux figures1 à 4, il peut être envisagé de remplacer la membrane incurvée par une membrane plane si celle-ci assure de manière satisfaisante la fonction consistant à tendre la pluralité de fibres capillaires sur la surface de réception 12.

On peut également envisager que des éléments décrits en référence à un des deux modes de réalisation puisse être utilisé dans l'autre mode de réalisation, par exemple, que la pluralité de fentes d'alignement 16.1 décrites en référence au premier mode de réalisation soient utilisées à la place du peigne décrit en référence au deuxième mode de réalisation ou inversement.

## Revendications

1. Dispositif de détermination de brillance configuré pour déterminer une brillance relative d'une pluralité de fibres capillaires, ou plus particulièrement d'une mèche de cheveux, dans lequel le dispositif de détermination de brillance comprend :
- une surface de réception (12) configurée pour recevoir la pluralité de fibres capillaires, ou plus particulièrement la mèche de cheveux ;
- un élément optique de mesure (14) configuré pour déterminer la brillance relative des fibres capillaires reçues sur la surface de réception (12);
- un élément d'alignement (16) configuré pour aligner la pluralité de fibres capillaires, ou plus particulièrement la mèche de cheveux, lorsque la pluralité de fibres capillaires, ou plus particulièrement la mèche de cheveux, est positionnée sur la surface de réception (12);
- un élément de mise sous tension (18) configuré pour mettre sous tension la pluralité de fibres capillaires, ou plus particulièrement la mèche de cheveux, sur ladite surface de réception (12);
- un élément d'occultation (20) configuré pour occulter au moins partiellement l'élément optique de mesure (14) de la lumière ambiante lorsque la pluralité de fibres capillaires, ou plus particulièrement la mèche de cheveux, est positionnée sur la surface de réception (12), **caractérisé en ce que** l'élément d'occultation (20) est mobile entre une position ouverte,
dans laquelle l'élément optique de mesure (14) n'est pas protégé de la lumière ambiante par l'élément d'occultation (20), et une position fermée, dans laquelle l'élément optique de mesure (14) est protégé de la lumière ambiante par l'élément d'occultation (20).

2. Dispositif de détermination de brillance selon la revendication 1, dans lequel l'élément de mise sous tension (18) est disposé sur l'élément d'occultation (20).

3. Dispositif de détermination de brillance selon l'une quelconque des revendications 1 ou 2, dans lequel l'élément d'occultation (20) est mobile en rotation autour d'un axe de rotation (AR).

4. Dispositif de détermination de brillance selon l'une quelconque des revendications 1 à 3, dans lequel le dispositif de détermination de brillance comprend un élément d'actionnement (22) mobile entre une première position et une deuxième position, l'élément d'actionnement (22) étant configuré pour entrainer un déplacement de l'élément d'occultation (20) entre la position fermée et la position ouverte lorsque l'élément d'actionnement (22) est déplacé entre la première position et la deuxième position.

5. Dispositif de détermination de brillance selon la revendication 4, dans lequel l'élément d'actionnement (22) comprend au moins une crémaillère (24) configurée pour coopérer avec au moins un secteur de roue dentée (26) prévue sur l'élément d'occultation (20).

6. Dispositif de détermination de brillance selon l'une quelconque des revendications 1 à 5, dans lequel le dispositif de détermination de brillance comprend un élément de rappel (30) configuré pour rappeler l'élément d'occultation (20) vers la position fermée.

7. Dispositif de détermination de brillance selon l'une quelconque des revendications 1 à 6, dans lequel le dispositif de détermination de brillance comprend un élément de blocage (32) configuré pour bloquer l'élément d'occultation (20) en position ouverte.

8. Dispositif de détermination de brillance selon l'une quelconque des revendications 1 à 7, dans lequel l'élément d'occultation (20) est amovible.

9. Dispositif de détermination de brillance selon la revendication 8, dans lequel le dispositif de détermination de brillance comprend un corps principal (10) sur lequel est monté l'élément optique de mesure (14), l'élément d'occultation (20) comprenant un premier élément de fixation (34) configuré pour coopérer avec un deuxième élément de fixation (36) prévu sur le corps principal (10) de sorte que ledit élément d'occultation (20) soit fixé de manière amovible au corps principal (10).

10. Dispositif de détermination de brillance selon l'une quelconque des revendications 8 ou 9, dans lequel l'élément d'occultation (20) comprend un premier aimant de fixation (38) configuré pour coopérer avec un deuxième aimant de fixation (40) de sorte que ledit élément d'occultation (20) soit fixé au reste du dispositif de détermination de brillance magnétiquement.

11. Dispositif de détermination de brillance selon l'une quelconque des revendication 1 à 10, dans lequel l'élément d'alignement (16) est un peigne configuré pour recevoir et aligner la pluralité de fibres capillaires, ou plus particulièrement la mèche de cheveux ou l'élément d'alignement (16) comprend au moins une fente d'alignement configurée pour recevoir et aligner la pluralité de fibres capillaires, ou plus particulièrement la mèche de cheveux.

12. Dispositif de détermination de brillance selon la revendication 11, dans lequel le dispositif de détermination de brillance comprend un canal d'insertion (17) configuré pour faciliter l'insertion de la pluralité de fibres capillaires dans l'au moins une fente d'alignement.

13. Dispositif de détermination de brillance selon l'une quelconque des revendications 1 à 12, dans lequel l'élément de mise sous tension (18) comprend au moins un patin de maintien.

14. Dispositif de détermination de brillance selon la revendication 13, dans lequel l'élément de mise sous tension (18) comprend deux patins de maintien disposés de part et d'autre de l'élément optique de mesure (14) lorsque l'élément d'occultation (20) est dans une position fermée.

15. Dispositif de détermination de brillance selon l'une des revendications 1 à 12, dans lequel l'élément de mise sous tension (18) comprend une membrane incurvée.

16. Appareil de traitement de fibres capillaires, ou plus particulièrement d'une mèche de cheveux, comprenant un dispositif de détermination de brillance selon l'une quelconque des revendications 1 à 15.

## Patentansprüche

1. Vorrichtung zur Bestimmung der Helligkeit, die konfiguriert ist, um eine relative Helligkeit einer Vielzahl von Haarfasern oder insbesondere einer Haarsträhne zu bestimmen, wobei die Vorrichtung zur Bestimmung der Helligkeit Folgendes umfasst:
- eine Aufnahmefläche (12), die konfiguriert ist, um die Vielzahl von Haarfasern oder insbesondere die Haarsträhne aufzunehmen;
- ein optisches Messelement (14), das konfiguriert ist, um die relative Helligkeit der auf der Aufnahmefläche (12) aufgenommenen Haarfasern zu bestimmen;
- ein Ausrichtungselement (16), das konfiguriert ist, um die Vielzahl von Haarfasern oder insbesondere die Haarsträhne auszurichten, wenn die Vielzahl von Haarfasern oder insbesondere die Haarsträhne auf der Aufnahmefläche (12) positioniert ist;
- ein Spannelement (18), das konfiguriert ist, um an der Vielzahl von Haarfasern oder insbesondere an der Haarsträhne auf der Aufnahmefläche (12) Spannung anzulegen;
- ein Verdeckungselement (20), das konfiguriert ist, um das optische Messelement (14) mindestens teilweise vor dem Umgebungslicht zu verdecken, wenn die Vielzahl von Haarfasern oder insbesondere die Haarsträhne auf der Aufnahmefläche (12) positioniert ist, **dadurch gekennzeichnet, dass** das Verdeckungselement (20) zwischen einer offenen Position, bei der das optische Messelement (14) nicht durch das Verdeckungselement (20) vor dem Umgebungslicht geschützt wird, und einer geschlossenen Position, bei der das optische Messelement (14) durch das Verdeckungselement (20) vor dem Umgebungslicht geschützt wird, beweglich ist.

2. Vorrichtung zur Bestimmung der Helligkeit nach Anspruch 1, wobei das Spannelement (18) auf dem Verdeckungselement (20) angeordnet ist.

3. Vorrichtung zur Bestimmung der Helligkeit nach einem der Ansprüche 1 oder 2, wobei das Verdeckungselement (20) um eine Drehachse (AR) herum drehbeweglich ist.

4. Vorrichtung zur Bestimmung der Helligkeit nach einem der Ansprüche 1 bis 3, wobei die Vorrichtung zur Bestimmung der Helligkeit ein Betätigungselement (22) umfasst, das zwischen einer ersten Position und einer zweiten Position beweglich ist, wobei das Betätigungselement (22) konfiguriert ist, um eine Verschiebung des Verdeckungselements (20) zwischen der geschlossenen Position und der offenen Position herbeizuführen, wenn das Betätigungselement (22) zwischen der ersten Position und der zweiten Position verschoben wird.

5. Vorrichtung zur Bestimmung der Helligkeit nach Anspruch 4, wobei das Betätigungselement (22) mindestens ein Zahngestänge (24) umfasst, das konfiguriert ist, um mit mindestens einem Sektor eines gezahnten Rads (26) zusammenzuwirken, das auf dem Verdeckungselement (20) vorgesehen ist.

6. Vorrichtung zur Bestimmung der Helligkeit nach einem der Ansprüche 1 bis 5, wobei die Vorrichtung zur Bestimmung der Helligkeit ein Rückstellelement (30) umfasst, das konfiguriert ist, um das Verdeckungselement (20) in die geschlossene Position zurückzustellen.

7. Vorrichtung zur Bestimmung der Helligkeit nach einem der Ansprüche 1 bis 6, wobei die Vorrichtung zur Bestimmung der Helligkeit ein Sperrelement (32) umfasst, das konfiguriert ist, um das Verdeckungselement (20) in der offenen Position zu arretieren.

8. Vorrichtung zur Bestimmung der Helligkeit nach einem der Ansprüche 1 bis 7, wobei das Verdeckungselement (20) abnehmbar ist.

9. Vorrichtung zur Bestimmung der Helligkeit nach Anspruch 8, wobei die Vorrichtung zur Bestimmung der Helligkeit einen Hauptkörper (10) umfasst, auf dem das optische Messelement (14) angebracht ist, wobei das Verdeckungselement (20) ein erstes Befestigungselement (34) umfasst, das konfiguriert ist, um mit einem zweiten Befestigungselement (36), das auf dem Hauptkörper (10) vorgesehen ist, derart zusammenzuwirken, dass das Verdeckungselement (20) auf abnehmbare Weise am Hauptkörper (10) befestigt wird.

10. Vorrichtung zur Bestimmung der Helligkeit nach einem der Ansprüche 8 oder 9, wobei das Verdeckungselement (20) einen ersten Befestigungsmagneten (38) umfasst, der konfiguriert ist, um mit einem zweiten Befestigungsmagneten (40) derart zusammenzuwirken, dass das Verdeckungselement (20) am Rest der Vorrichtung zur Bestimmung der Helligkeit magnetisch befestigt wird.

11. Vorrichtung zur Bestimmung der Helligkeit nach einem der Ansprüche 1 bis 10, wobei das Ausrichtungselement (16) ein Kamm ist, der konfiguriert ist, um die Vielzahl von Haarfasern oder insbesondere die Haarsträhne aufzunehmen und auszurichten, oder wobei das Ausrichtungselement (16) mindestens einen Ausrichtungsspalt umfasst, der konfiguriert ist, um die Vielzahl von Haarfasern oder insbesondere die Haarsträhne aufzunehmen und auszurichten.

12. Vorrichtung zur Bestimmung der Helligkeit nach Anspruch 11, wobei die Vorrichtung zur Bestimmung der Helligkeit einen Einsatzkanal (17) umfasst, der konfiguriert ist, um das Einsetzen der Vielzahl von Haarfasern in dem mindestens einen Ausrichtungsspalt zu erleichtern.

13. Vorrichtung zur Bestimmung der Helligkeit nach einem der Ansprüche 1 bis 12, wobei das Spannelement (18) mindestens einen Haltegleiter umfasst.

14. Vorrichtung zur Bestimmung der Helligkeit nach Anspruch 13, wobei das Spannelement (18) zwei Haltegleiter umfasst, die beiderseits des optischen Messelements (14) angeordnet sind, wenn das Verdeckungselement (20) in einer geschlossenen Position ist.

15. Vorrichtung zur Bestimmung der Helligkeit nach einem der Ansprüche 1 bis 12, wobei das Spannelement (18) eine gekrümmte Membran umfasst.

16. Einrichtung zur Behandlung von Haarfasern oder insbesondere einer Haarsträhne, umfassend eine Vorrichtung zur Bestimmung der Helligkeit nach einem der Ansprüche 1 bis 15.

## Claims

1. Device for determining brightness configured to determine a relative brightness of a plurality of hair fibres, or more specifically, of a hair strand, wherein the device for determining brightness comprises:
- a receiving surface (12) configured to receive the plurality of hair fibres, or more specifically, the hair strand;
- an optical measuring element (14) configured to determine the relative brightness of hair fibres received on the receiving surface (12);
- an alignment element (16) configured to align the plurality of hair fibres, or more specifically, the hair strand, when the plurality of hair fibres, or more specifically the hair strand, is positioned on the receiving surface (12);
- a tensioning element (18) configured to tension the plurality of hair fibres, or more specifically the hair strand, on said receiving surface (12);
- a concealing element (20) configured to conceal at least partially the optical measuring element (14) from ambient light when the plurality of hair fibres, or more specifically the hair strand, is positioned on the receiving surface (12), **characterised in that** the concealing element (20) is movable between an open position, wherein the optical measuring element (14) is not protected from ambient light by the concealing element (20), and a closed position, wherein the optical measuring element (14) is protected from ambient light by the concealing element (20).

2. Device for determining brightness according to claim 1, wherein the tensioning element (18) is disposed on the concealing element (20).

3. Device for determining brightness according to any one of claims 1 or 2, wherein the concealing element (20) is rotatably movable about an axis of rotation (AR).

4. Device for determining brightness according to any one of claims 1 to 3, wherein the device for determining brightness comprises an actuation element (22) which is movable between a first position and a second position, the actuation element (22) being configured to drive a movement of the concealing element (20) between the closed position and the open position when the actuation element (22) is moved between the first position and the second position.

5. Device for determining brightness according to claim 4, wherein the actuation element (22) comprises at least one rack (24) configured to engage with at least one toothed wheel sector (26) provided on the concealing element (20).

6. Device for determining brightness according to any one of claims 1 to 5, wherein the device for determining brightness comprises a return element (30) configured to return the concealing element (20) to the closed position.

7. Device for determining brightness according to any one of claims 1 to 6, wherein the device for determining brightness comprises a blocking element (32) configured to block the concealing element (20) in the open position.

8. Device for determining brightness according to any one of claims 1 to 7, wherein the concealing element (20) is removable.

9. Device for determining brightness according to claim 8, wherein the device for determining brightness comprises a main body (10) on which is mounted the optical measuring element (14), the concealing element (20) comprising a first fixing element (34) configured to engage with a second fixing element (36) provided on the main body (10), such that said concealing element (20) is removably fixed to the main body (10).

10. Device for determining brightness according to any one of claims 8 or 9, wherein the concealing element (20) comprises a first fixing magnet (38) configured to engage with a second fixing magnet (40), such that said concealing element (20) is fixed to the rest of the device for determining brightness magnetically.

11. Device for determining brightness according to any one of claims 1 to 10, wherein the alignment element (16) is a comb configured to receive and align the plurality of hair fibres, or more specifically the hair strand or the alignment element (16) comprises at least one alignment slot configured to receive and align the plurality of hair fibres, or more specifically, the hair strand.

12. Device for determining brightness according to claim 11, wherein the device for determining brightness comprises an insertion channel (17) configured to facilitate the insertion of the plurality of hair fibres into the at least one alignment slot.

13. Device for determining brightness according to any one of claims 1 to 12, wherein the tensioning element (18) comprises at least one support slider.

14. Device for determining brightness according to claim 13, wherein the tensioning element (18) comprises two support sliders disposed on either side of the optical measuring element (14) when the concealing element (20) is in a closed position.

15. Device for determining brightness according to one of claims 1 to 12, wherein the tensioning element (18) comprises a curved membrane.

16. Appliance for treating hair fibres, or more specifically a hair strand, comprising a device for determining brightness according to any one of claims 1 to 15.
